(19) Europäisches Patentamt

European Patent Office

Office européen des brevets

(11) EP 1 201 679 A2

(12) EUROPEAN PATENT APPLICATION

(43) Date of publication:
02.05.2002 Bulletin 2002/18

(51) Int Cl.⁷: **C07K 4/08**, C07K 1/12,
A23L 1/305
// (C07K4/08, 123:00)

(21) Application number: 01125636.9

(22) Date of filing: 26.10.2001

(84) Designated Contracting States:
AT BE CH CY DE DK ES FI FR GB GR IE IT LI LU
MC NL PT SE TR
Designated Extension States:
AL LT LV MK RO SI

(30) Priority: 27.10.2000 JP 2000329566

(71) Applicant: RIKEN VITAMIN CO., LTD.
Chiyoda-ku, Tokyo (JP)

(72) Inventors:
• Sato, Minoru
Aoba-ku, Sendai-shi, Miyagi-ken (JP)
• Ooba, Takashi
Aoba-ku, Sendai-shi, Miyagi-ken (JP)

• Kobayashi, Akio
1-15-10, Wakagi, Itabashi-ku, Tokyo (JP)
• Funayama, Katsura
1-15-10, Wakagi, Itabashi-ku, Tokyo (JP)
• Kahara, Takashi
Chiyoda-ku, Tokyo (JP)
• Nakano, Takahisa
Chiyoda-ku, Tokyo (JP)

(74) Representative:
TER MEER STEINMEISTER & PARTNER GbR
Patentanwälte,
Mauerkircherstrasse 45
81679 München (DE)

(54) **Angiotensin I-converting enzyme inhibitory substance**

(57) The subject of the invention is to produce a peptide with potent the angiotensin I-converting enzyme (ACE) inhibitory activity by decomposing the protein contained in wakame with protease, using wakame being a food material ingested daily as a raw material, and to provide a safe food composition with hypotensive activity, using the treatment product thereof as a raw material.

The inventors have found that, when performing the decomposing treatment of wakame with various proteases, a group of endo-type enzymes produced by Bacillus produce peptides with particularly strong ACE inhibitory activity, leading to the completion of the invention.

**Description**

BACKGROUND OF THE INVENTION

The present invention relates to an angiotensin I-converting enzyme (hereinafter, abbreviated as ACE) inhibitory substance with blood-pressure lowering activity, in more detail, peptide with potent ACE inhibitory activity obtained by decomposing wakame (Undaria pinnatifida) (a kind of brown seaweed family) with protease.

The hypertension is one of the causes liable to induce the arterial sclerosis together with hyperlipidemia, diabetes and obesity. The population diagnosed as being hypertension is presumed to be twenty millions or more in Japan, and the potential hypertension is also said to run to a considerable number. In recent years, the diagnostic criterion for the hypertension has become severe, but the hypertension is said to be a silent killer. Though symptomless, if leaving it as it is, it often incurs the death, hence it is one of the diseases of which the life habit is said to be important. For improving this hypertension, the development of hypotensor drugs and food components for regulating blood pressure is being put forward positively at many research organizations.

In the organism, the renin-angiotensin system is an important factor that regulates blood pressure and the balance of quantity of water in body with electrolytes. The renin secreted from jaxtaglomerular cells of kidney into circulating blood acts on the angiotensinogen being biosynthesized in liver and existing in blood to make angiotensin I. The angiotensin I becomes angiotensin II with ACE in the endothelial cell of blood vessel. The angiotensin II has three major physiological actions; ① it contracts the smooth muscle of blood vessel very strongly to increase the blood pressure, ② it promotes the secretion of aldosterone from adrenal cortex into blood, and ③ it acts on the proximal tubules of kidney to enhance the reabsorption of sodium filtered through glomerulus. All of these actions work so as to increase the blood pressure. In addition, the ACE decomposes bradykinin having an action to dilate the smooth muscle of blood vessel to inactivate it. As described, the ACE has the activation of renin-angiotensin system (hypertensive system) and the inactivation of kallikrein-kinin system (hypotensive system) at the same time and, as a result, it exhibits the action to increase the blood pressure. Hence, a substance that inhibits the activity of ACE can suppress an increase in the blood pressure, thus, medicinal drugs and foods containing inhibitory substance are being developed, focusing thereon.

As a substance with potent ACE inhibitory activity using food material as a raw material, it is known that peptide obtained by decomposing the protein of animals and plants with a certain kind of protease is effective. As the peptides with potent ACE inhibitory activity are producible with protease, those using raw materials from animals and plants such as tuna (Patent Publication No. 2049147), sap of fig tree (Patent Publication No. 2794094), antarctic krill (Patent Publication No. 2088612), soy protein (Patent Publication No. 1976328), sardine (Patent Publication No. 2086032), casein (Patent Publication No. 1814531), dried bonito (Kokai No. Hei 04-69397), gluten (Kokai No. Hei 04-66594) and rice protein (Kokai No. Hei 04-279529) are disclosed. Thereamong, however, substances put into practice are very few. For the reasons, it is mentioned that insufficient effect and taste and odor make it difficult to be used for foods, and the like. Moreover, those using raw materials from seaweeds such as laver (Patent Publication No. 2678180) and hijiki (Kokai No. Hei 10-36391) are disclosed, and the applicant has also confirmed the ACE inhibitory effect as for four kinds of tetrapeptides obtained by hydrolyzing wakame (Patent Application No. Hei 11-284647). Furthermore, a health food containing enzymolysis product of seaweed (Kokai No. Hei 07-289202) is also disclosed. These inventions using laver, hijiki and wakame as raw materials use pepsin in all cases as a protein hydrolase to obtain peptides with potent ACE inhibitory activity.

In the side of science or medicinal drug, it is important to identify the physiologically active substance and use it after isolation and purification, but, when considering the utilization as a food, the use thereof after its isolation and purification is not necessarily practical in the industry from the points of economics etc. When utilizing as a food composition, a crude fraction or lightly purified fraction of decomposition product of protein is actually practical in many cases. Moreover, the products obtained by hydrolyzing protein using pepsin as a protease have bad taste for utilizing as foods and further the blood-pressure lowering activity of crude fraction or lightly purified fraction thereof is not necessarily effective.

Moreover, in Kokai No. Hei 07-289202, a method of obtaining peptide by hydrolyzing Kombu (Laminaria) with alkalase is disclosed, but there is no description on blood pressure in this patent.

A superaging society has come in Japan and advanced nations and increased medical expenses has become a serious problem. Moreover, it is also a problem that, though the average life span is extended, aged persons who cannot spend a healthy social life because of being bedridden or dementia are increasing. From the viewpoint of improving such problems, a way of thinking to perform the maintenance and promotion of health with daily foods is now penetrating.

SUMMARY OF THE INVENTION

The subject of the invention is to produce a peptide with potent ACE the inhibitory activity by decomposing the protein of wakame with protease, using wakame being a food material ingested daily as a raw material, and to provide a safe food composition with blood-pressure lowering activity, using the treatment product thereof as a raw material.

In view of said subject, the inventors have found that, when performing the decomposing treatment of wakame with various proteases, a group of endo-type enzymes produced by Bacillus produce peptides with potent particularly strong ACE inhibitory activity, leading to the completion of the invention.

DETAILED DESCRIPTION OF THE INVENTION

The proteases to be used in the invention are endo-type enzymes. The decomposition products obtained by hydrolyzing with exo-type enzymes show only weak ACE inhibitory activity.

As the endo-type enzymes, enzymes produced by Bacillus, preferably Bacillus subtilis and Bacillus stearothermophilus, are used. When performing the decomposing treatment of wakame with these protein hydrolases, a group of peptides with potent particularly strong ACE inhibitory activity can be produced, and, with papain, blomelain, pancreatin, Aspergillus enzymes and Rhizopus enzymes being other publicly known proteases, production of the group of peptides with strong ACE inhibitory activity as shown in the invention cannot be obtained.

Although the ACE inhibitory activity of decomposition product with pepsin described in Kokai No. Hei 11-284647 falls under the strong category, the extent of action thereof is weaker than that with said enzymes according to the invention, and further the pepide of peptic digest of wakame have a bitter taste.

Moreover, even if said enzymes according to the invention may be used, when the kind of raw material seaweed differs, the peptides with as strong ACE inhibitory activity as wakame cannot be obtained and, in some cases, the ACE inhibitory activity cannot be obtained at all. Namely, it can be said that, for the inventive enzymes, the protein of wakame is a preferable substrate to produce the substances with ACE inhibitory activity.

When decomposing the wakame with the inventive proteases, it is preferable to conduct at the optimum temperature and optimum pH value of those enzymes from the aspects of shorting of reaction time and stability of enzyme, but, if being under conditions within a common-sense range, the objects can be obtained. Moreover, even if both enzymes produced with Bacillus subtilis and Bacillus stearothermophilus may be reacted in sequence, the peptides with strong ACE inhibitory activity can be obtained.

With respect to the wakame to be used as a raw material, all portions of leaf, stalk and sporophyll of wakame may be all right. Moreover, with respect to the leaf of wakame, any of dried wakame, boiled and salt-cured wakame, salt-cured wakame and raw wakame may be all right, and the thickness of leaf, color or habitat is not minded. Namely, irrespective of the habitat of raw material wakame and its shape, the peptides with equal ACE inhibitory activity can be obtained.

Moreover, in order to simplify manufacturing process and make efficient decomposition rate of protein into wakame, it is preferable that alginic acid exists in wakame remove by using of alginic acid lyase at first step of the process, it is expedient to obtain the peptides of wakame. Generally, in the case of wakame being in aqueous solution more than pH 4, the alginic acid into wakame dissolves out, hence the viscosity of solution is very high. Such high viscosity solution is different to stir, separate to solid-liquid and ultrafilter. By pretreatment with alginic acid lyase, alginic acid is degraded to low-molecular saccharide. In that case, the viscosity of solution decreases, leading to improved workability. Even if it uses alginic acid lyase, there is not change of ACE inhibitory activity.

Since the peptides obtained in the invention have less specific unpleasantness in taste and odor, it is preferable to ingest them by oral administration. The dose differs depending on the age and the extent of blood pressure, but it is usually 10mg to 2000mg at once and the effect is obtained once to thrice a day.

The wakame peptides obtained in the invention can be administered by themselves or after mixed appropriately with excipient etc. for the convenience of pharmacy to convert to the shapes of powder, granule, tablet, capsule, etc. Also, it is possible to ingest by formulating or mixing with various foods such as candy, jelly, tablet cake, beverage, soup, noodles, rice cracker, Japanese cake, cold cake and baked cake.

In following, the invention will be illustrated in detail based on examples.

<Example 1>

Flaky dried wakame was pulverized with ultracentrifugal pulverizer to make the particle diameter 0.2mm or less and 900g of this powder were suspended and dispersed into 18kg of water. Then, 0.09g of alginic acid lyase (from Nagase Biochemical Industries Co.) were added thereto and the mixture was treated for 18 hours at 45°C. The treated product was centrifuged for 5 minutes at 5000rpm. The precipitates were washed with water, dried and then pulverized to obtain a sample for treating with protease.

Into 25ml of water was suspended and dispersed 1g of this sample and, after 10mg of each protease were added, the decomposing treatment with enzyme was performed for 18 hours at a fixed temperature and pH condition. For the adjustment of pH value, sodium hydroxide and hydrochloric acid were used. After decomposition with enzyme, the product was boiled for 15 minutes at 100°C to inactivate the enzyme. The precipitates were removed by centrifugation at 100,000rpm for 10minutes to obtain a supernatant. This supernatant were ultrafiltered (Ultra Free-MC, from Millipore Corp., fractional molecular weight 10,000) and were used assay for ACE inhibitory activity.

The ACE inhibitory activity was determined by following method.

[Method of determining the ACE inhibitory activity]

Substrate solution:

12.5mM Hippuryl-L-histidinyl-L-leucine (from Sigma Corp.)
A solution of 25mg of hippuryl-L-histidinyl-L-leucine dissolved into 4.6ml of boric acid buffer at pH 8.3.

Solution of ACE:

250mU/ml ACE (from Sigma Corp.)
A solution of 0.25U of ACE        dissolved into 1ml of boric acid buffer at pH 8.3.

Into a test tube were taken 35μl of protease-treated solution obtained by ultrafiltration, and 100μl of substrate solution and 35μl of solution of angiotensin-transforming enzyme were added, which was reacted for 1 hour at 37°C. Then, 125μl of 0.5N hydrochloric acid were added to stop the reaction, and 2ml of ethyl acetate were added, followed by violent shaking. The solution was centrifuged for 10 minutes at 2,500rpm and 1.5ml of ethyl acetate layer was sampled. After ethyl acetate was removed from this ethyl acetate layer under reduced pressure while replacing with nitrogen, 700μl of 1M NaCl solution were added and the quantity of hippuric acid extracted was measured from the absorbance at 228nm to obtain the enzyme activity. The ACE inhibitory activity was calculated from following formula:

$$\text{ACE Inhibitory activity (\%)} = (A-B)/A \times 100$$

A: Absorbance when ACE inhibitor is not contained
B: Absorbance when ACE inhibitor is added

The activity of an ACE inhibitory content was expressed as the amount needed to inhibit 50% of ACE activity (IC50) under these conditions.

Table 1 shows 18 kinds of enzymes offered to test and their origins, and Table 2 shows the conditions of enzyme treatment (temperature and pH value) and the ACE inhibitory activity (%).

Besides, with respect to Protease S " Amano", Protease N " Amano" and Proleather FG-F, which exhibited ACE inhibitory activity effectively, tests of two-stage reaction of treatment with Protease S "Amano" followed by treatment with Proleather FG-F (Sample No. 20), and two-stage reaction of treatment with Protease S "Amano" followed by treatment with Protease N "Amano" (Sample No. 21) were performed, respectively.

Table 1:

| Proteases offered to Example 1 | | | |
|---|---|---|---|
| No. of sample | Name of product | Manufacturing company | Origin of enzyme |
| 1 | Protease S "Amano" | Amano Pharmaceutical | Bacillus stearothermophilus |
| 2 | Bioblase SP-15 FG | Nagase Biochemical Industries | Bacillus subtilis |
| 3 | Proleather FG-F | Amano Pharmaceutical | Bacillus subtilis |
| 4 | Protease N "Amano" | Amano Pharmaceutical | Bacillus subtilis |
| 5 | Pepsin | Amano Pharmaceutical | Animal stomach |
| 6 | Denapsin | Nagase Biochemical Industries | Aspergillus niger |
| 7 | Newlase F | Amano Pharmaceutical | Rhizopus niveus |
| 8 | Protease P "Amano" 3G | Amano Pharmaceutical | Aspergillus mellens |
| 9 | Protease M "Amano" | Amano Pharmaceutical | Aspergillus oryzae |

Table 1: (continued)

| Proteases offered to Example 1 | | | |
|---|---|---|---|
| No. of sample | Name of product | Manufacturing company | Origin of enzyme |
| 10 | XP-415 | Nagase Biochemical Industries | Rhizopus delemar |
| 11 | Umamizyme | Amano Pharmaceutical | Aspergillus oryzae |
| 12 | Protease A "Amano" G | Amano Pharmaceutical | Aspergillus oryzae |
| 13 | Pancreatine F | Amano Pharmaceutical | Animal panreas |
| 14 | Alkalase 2.4L FG | Nobonordisk Bioindustry | Bacillus licheniformis |
| 15 | Papain W-40 | Amano Pharmaceutical | Carica papaya L. |
| 16 | Denazyme AP | Nagase Biochemical Industries | Aspergillus oryzae |
| 17 | Blomelain F | Amano Pharmaceutical | Pineapple cannery |
| 18 | Purified papain for foods | Nagase Biochemical Industries | Carica L. |
| 19 | Peptidase R | Amano Pharmaceutical | Rhizopus oryzae |
| 20 | Protease S "Amano" → Proleather FG-F | | |
| 21 | Protease S "Amano" → Protease N "Amano" | | |

Table 2:

| No. of sample | Test conditions | | Inhibitory activity (%) |
|---|---|---|---|
| | Temperature (°C) | pH | |
| 1 | 70 | 8.0 | 91.0 |
| 2 | 65 | 10.0 | 83.6 |
| 3 | 60 | 10.0 | 82.2 |
| 4 | 55 | 7.0 | 76.8 |
| 5 | 45 | 2.0 | 74.0 |
| 6 | 50 | 3.0 | 60.0 |
| 7 | 45 | 3.0 | 58.5 |
| 8 | 45 | 8.0 | 56.3 |
| 9 | 50 | 4.5 | 55.1 |
| 10 | 55 | 3.0 | 52.4 |
| 11 | 50 | 7.0 | 50.2 |
| 12 | 50 | 7.0 | 32.7 |
| 13 | 45 | 9.0 | 25.2 |
| 14 | 60 | 7.0 | 22.4 |
| 15 | 65 | 7.0 | 17.5 |
| 16 | 50 | 7.0 | 0 |
| 17 | 60 | 9.0 | 0 |
| 18 | 70 | 7.0 | 0 |
| 19 | 45 | 7.0 | 0 |
| 20 | 70→60 | 8.0→10.0 | 95.0 |

Test conditions of Example 1 and results of inhibitory activity on angiotensin-transforming enzyme

Table 2: (continued)

| Test conditions of Example 1 and results of inhibitory activity on angiotensin-transforming enzyme | | | |
|---|---|---|---|
| No. of sample | Test conditions | | Inhibitory activity (%) |
| | Temperature (°C) | pH | |
| 21 | 70→55 | 8.0→7.0 | 85.0 |

<Example 2>

After 100ml of water were added to 2g of flaky dried wakame to swell, this was washed with water. Then, 100ml of water and 20mg of protease were added and the mixture was submitted to enzymolysis treatment for 18 hours at a fixed temperature and pH condition. For the adjustment of pH value, sodium hydroxide and hydrochloric acid were used.

After enzymolysis, the product was boiled for 15 minutes at 100°C to inactivate the enzyme. After cooling by allowing to stand, this was centrifuged for 5 minutes at 20,000rpm to obtain a supernatant. Water was added to this supernatant and the volume was adjusted accurately to 150ml. Then, part of this was treated by ultrafiltration (Ultra Free-MC, from Millipore Corp., fractional molecular weight 10,000) to obtain a permeated solution. This permeated solution was diluted appropriately and the ACE inhibitory activity was determined to obtain IC50 value. The proteases offered to test, hydrolysis conditions and results are shown in Table 3.

Table 3:

| Proteases offered to Example 2 and inhibitory activity on angiotensin-transforming enzyme | | | |
|---|---|---|---|
| No. of enzyme sample offered (Table 1) | Test conditions | | Inhibitory activity (IC50) |
| | Temperature (°C) | pH | |
| 1 | 70 | 8.0 | 86 |
| 3 | 60 | 10.0 | 117 |
| 4 | 55 | 7.0 | 160 |
| 5 | 45 | 2.0 | 220 |

<Example 3>

To 200g of boiled and salt-cured wakame were added 3000ml of water and 3mg of alginic acid lyase, and the mixture was submitted to enzymolysis treatment for 18 hours at 45°C. After reaction, the product was centrifuged for 5 minutes at 5000rpm and the precipitates were washed with water, dried and then pulverized to obtain a sample. Each 1g of this sample was treated similarly to Example 1, using each 10mg of four kinds of proteases shown in Table 3 to determine the ACE inhibitory activity.

The respective inhibitory activities (%) were Protease S "Amano": 89%, Proleather FG-F: 84%, Protease N "Amano": 75% and pepsin: 72%, thus giving the same results as in Example 1 wherein dried wakame was used.

Each of the wakame peptides obtained by using Proleather FG-F, Protease S " Amano" and pepsin was given by single oral administration (forced administration by stomach sonde) at a dose of each 100mg/kg to male spontaneously hypertensive rats (SHR) of 11 weeks old (3 rats per group of plot, rats with systolic pressure of 180mmHg or higher were used), and the blood pressure was measured at 3, 6, 9 and 24 hours after administration. The blood pressures at the time of start and at 6 hours and 24 hours after administration are shown in Table 4.

Table 4:

| Results of Example 4 | | | |
|---|---|---|---|
| Plot | blood pressure (mmHg) | | |
| | At the time of start | At 6 hours after administration | At 24 hours after administration |
| Control (water) | 200.5 | 197.2 (-3.3) | 207.3 |
| Prorazor FG-F | 207.0 | 186.8 (-20.2) | 206.1 |

Table 4: (continued)

| Results of Example 4 | | | |
|---|---|---|---|
| Plot | blood pressure (mmHg) | | |
| | At the time of start | At 6 hours after administration | At 24 hours after administration |
| Protease S "Amano" | 201.3 | 180.1 (-21.2) | 204.4 |
| Pepsin | 206.2 | 196.0 (-10.2) | 215.2 |
| Note-1) Control plot: Number of rats=6 Note-2) Bracket in the column of "At 6 hours after administration" is changes in systolic blood pressure of SHR at 6hours after oral administration. | | | |

[Comparative example]

Replacing flaky dried wakame in Example 2 with powder of Kombu (Laminaria, brown seaweed family), similar treatment was performed. As a result, in all treatment products with proteases, no inhibitory activity on angiotensin-transforming enzyme was recognized.

In accordance with the invention, by using the wakame peptides obtained by decomposing wakame with endo-type enzymes originating from Bacillus, high-safety food materials with hypotensive activity can be obtained.

**Claims**

1. An angiotensin I-converting enzyme inhibitory substance, containing peptide obtained by decomposing wakame with protease other than pepsin.

2. The angiotensin I-converting enzyme inhibitory substance of Claim 1, wherein the protease is an endo-type protease originating from Bacillus.

3. A decomposition conduct of protein with blood-pressure lowering activity **characterized by** containing a peptide with potent an angiotensin I-converting enzyme inhibitory activity wherein said peptide is obtained by decomposing wakame with a protease other than pepsin.

4. A food composition employing the decomposition product of protein defined in claim 3 as a starting material thereof.

5. A preparing method of an angiotensin I-converting enzyme inhibitory substance, **characterized by** decomposing wakame with protease other than pepsin to give a peptide with potent an angiotensin I-converting enzyme inhibitory activity.

6. The preparing method of an angiotensin I-converting enzyme inhibitory substance defined in claim 5, wherein wakame is subject to a pretreatment with alginic acid lyase to remove alginic acid present in wakame and thus pretreated wakame is subjected to a decomposition with a protease other than pepsin to give a peptide with potent an angiotensin I-converting enzyme inhibitory activity.